# EUROPEAN PATENT SPECIFICATION

(11) **EP 3 179 971 B1**
(45) Date of publication and mention of the grant of the patent: **17.06.2020**
(21) Application number: 15827804.4
(22) Date of filing: 29.07.2015
(51) Int. Cl.: A61F 13/15, A61F 13/53, B32B 3/26

(54) **PULPLESS ABSORBENT CORE AND METHOD OF CORE FORMING**
ZELLSTOFFFREIER ABSORBIERENDER KERN UND VERFAHREN ZUR KERNFORMUNG
NOYAU ABSORBANT EXEMPT DE PÂTE À PAPIER ET PROCÉDÉ DE FORMATION DE NOYAU

(30) Priority: 30.07.2014 US 201462030911 P; 10.11.2014 US 201462077418 P
(43) Date of publication of application: 21.06.2017
(73) Proprietor: Curt G. Joa, Inc., Sheboygan Falls Wisconsin 53085 (US)
(72) Inventor: WEI, Wei, North Hollywood, CA 91601 (US); HOHM, Gottfried Jason, Sheboygan Falls, WI 53085 (US)
(74) Representative: Potter Clarkson
(86) International application number: PCT/US2015/042663
(87) International publication number: WO 2016/019018

(56) References cited:
- US-A- 5 248 524
- US-A- 5 429 788
- US-A1- 2003 120 243
- US-A1- 2005 101 929
- US-A1- 2006 167 424
- US-A1- 2006 184 149
- US-A1- 2007 148 433
- US-A1- 2014 155 853

## Description

### Background of the Invention

This invention relates to formation of pulpless absorbent cores for use in disposable products such as diapers and sanitary napkins.

Sanitary napkins used in feminine hygiene are absorbent items worn by women to recover undesirable bodily discharges. These absorbent articles are typically comprised of an absorbent core sandwiched between layers of woven or non-woven materials.

Generally, diapers comprise an absorbent insert or patch and a chassis, which, when the diaper is worn, supports the insert proximate a wearer's body. Additionally, diapers may include other various patches, such as tape tab patches, reusable fasteners and the like. The raw materials used in forming a representative insert are typically cellulose pulp, tissue paper, poly, nonwoven web, acquisition, and elastic, although application specific materials are sometimes utilized. Usually, most of the insert raw materials are provided in roll form, and unwound and applied in assembly line fashion.

In the creation of a diaper (and, oftentimes also in conjunction with feminine hygiene products), multiple roll-fed web processes are typically utilized.

To create an absorbent insert, the cellulose pulp is unwound from the provided raw material roll and pulverized by a pulp mill. Discrete pulp cores are formed by a core forming assembly and placed on a continuous tissue web. Optionally, super-absorbent powder or polymer (SAP) may be added to the pulp core. The tissue web is wrapped around the pulp core. The wrapped core is debulked by proceeding through a calendar unit, which at least partially compresses the core, thereby increasing its density and structural integrity. After debulking, the tissue-wrapped core is passed through a segregation or knife unit, where individual wrapped cores are cut. The cut cores are conveyed, at the proper pitch, or spacing, to a boundary compression unit.

While the insert cores are being formed, other insert components are being prepared to be presented to the boundary compression unit. For instance, the poly sheet is prepared to receive a cut core. Like the cellulose pulp, poly sheet material is usually provided in roll form. The poly sheet is fed through a splicer and accumulator, coated with an adhesive in a predetermined pattern, and then presented to the boundary compression unit. In addition to the poly sheet, which may form the bottom of the insert, a two-ply top sheet may also be formed in parallel to the core formation. Representative plies are an acquisition web material and a nonwoven web material, both of which are fed from material rolls, through a splicer and accumulator. The plies are coated with adhesive, adhered together, cut to size, and presented to the boundary compression unit. Therefore, at the boundary compression unit, three components are provided for assembly: the poly bottom sheet, the core, and the two-ply top sheet.

A representative boundary compression unit includes a die roller and a platen roller. When all three insert components are provided to the boundary compression unit, the nip of the rollers properly compresses the boundary of the insert. Thus, provided at the output of the boundary compression unit is a string of interconnected diaper inserts. The diaper inserts are then separated by an insert knife assembly and properly oriented. At this point, the completed insert is ready for placement on a diaper chassis.

A representative diaper chassis comprises nonwoven web material and support structure. The diaper support structure is generally elastic and may include leg elastic, waistband elastic and belly band elastic. The support structure is usually sandwiched between layers of the nonwoven web material, which is fed from material rolls, through splicers and accumulators. The chassis may also be provided with several patches, besides the absorbent insert. Representative patches include adhesive tape tabs and resealable closures.

The process utilizes two main carrier webs; a nonwoven web which forms an inner liner web, and an outer web that forms an outwardly facing layer in the finished diaper. In a representative chassis process, the nonwoven web is slit at a slitter station by rotary knives along three lines, thereby forming four webs. One of the lines is on approximately the centerline of the web and the other two lines are parallel to and spaced a short distance from the centerline. The effect of such slicing is twofold; first, to separate the nonwoven web into two inner diaper liners. One liner will become the inside of the front of the diaper, and the second liner will become the inside of the back of that garment. Second, two separate, relatively narrow strips are formed that may be subsequently used to cover and entrap portions of the leg-hole elastics. The strips can be separated physically by an angularly disposed spreader roll and aligned laterally with their downstream target positions on the inner edges of the formed liners.

After the nonwoven web is sliced, an adhesive is applied to the liners in a predetermined pattern in preparation to receive leg-hole elastic. The leg-hole elastic is applied to the liners and then covered with the narrow strips previously separated from the nonwoven web. Adhesive is applied to the outer web, which is then combined with the assembled inner webs having elastic thereon, thereby forming the diaper chassis. Next, after the elastic members have been sandwiched between the inner and outer webs, an adhesive is applied to the chassis. The chassis is now ready to receive an insert.

To assemble the final diaper product, the insert must be combined with the chassis. The placement of the insert onto the chassis occurs on a placement drum or at a patch applicator. The inserts are provided to the chassis on the placement drum at a desired pitch or spacing. The generally flat chassis/insert combination is then folded so that the inner webs face each other, and the combination is trimmed. A sealer bonds the webs at appropriate locations prior to individual diapers being cut from the folded and sealed webs.

Generally, disposable undergarments such as pants-type diapers are made up of two nonwoven layers of material with elastic strands of material placed between the two nonwoven layers of material thus creating an elastic web laminate. The layers of material are continuous sheets of material that are eventually cut into individual undergarment lengths. The elastic strands may be arranged and cut so that specific areas of the undergarment are free of elastic tension or forces. An absorbent pad, often contained within an insert or core is then also placed into the pants-type diaper product.

To insure the pants-type diaper retains a proper shape and to hold all of the added layers of the diaper, reinforcing layers and backing materials are normally added to the continuous sheets of material, with the reinforcing layers corresponding to the cut elastic strands of each individual blank. Each of these layers needs to be adhesively joined at some point in the manufacturing process to the elastic web laminate to form the completed undergarment.

Often, void spaces need to be created in the diaper, such as holes cut out of the main web for provided leg holes when the undergarment is ultimately formed. To create the void spaces, the web is ordinarily die cut, with the web severed between a die and an anvil. The portion of the web material that is removed is referred to as a "chip." As the die wears throughout time, the severing of the chip from the web material becomes gradually a duller cut. This complicates the removal of the chip because the severing might not create a continuous cut out chip, with possibly some strands of the web material still coupling the chip with the web. It is desired to lengthen the amount of time and increase the number of chips that a single die can effectively be used for, to reduce the number of die change-outs.

Typically, the absorbent fibrous material is composed of cellulose wadding or cellulosic wood pulp material commonly referred to as "fluff", although a mixture of natural and synthetic fibers is within the scope of the invention. An absorbent core composed of wood pulp fluff is typically formed by employing conventional air laying techniques.

These absorbent cores have had their total absorbency improved greatly by the addition of super absorbent material, or super absorbent polymer (SAP) to the commonly used absorbent fibrous materials.

The ability of these absorbent cores to manage the typical surges of liquid flow is heavily dependent on the proper distribution of super absorbent material within the absorbent fluff. When most super absorbent materials absorb aqueous fluids, they swell substantially, often to double their dry dimensions or more at saturation. As these super absorbent materials absorb fluid and swell, they generally become a gelatinous mass.

There has been a trend in reducing the bulk of diapers, in attempts to make them more like underwear and to take up less shelf space in retailer's outlets. Generally, the thinner the diaper, the higher the concentration of super absorbent material required to produce the same level of absorbency. High levels of super absorbent material, however, tend to be more difficult to control and to maintain in position.

In conventional core forming processes, three-dimensional fluff receiving pockets rotate about a vacuum drum. The pockets typically include baffles and screens which permit airflow through the pockets. The fluff is applied to the fluff receiving pockets entrained in air applied to the pockets. The vacuum attracts the fluff to a screen-like mesh that forms the pockets. The fluff is retained by the pockets, and the amount of fluff builds up from the screen forming the pocket. However, some fluff passes through the screen of the pockets and into the vacuum stream that is drawing the fluff into the pocket. Thus, some fluff undesirably becomes entrained in the vacuum stream.

In conventional core forming process, it is desired to balance the amount of air urging the fluff towards the core forming pocket and the amount of vacuum used to retain the fluff within the pocket. Machine processes have become more complex as speeds of machines have increased, so air handling systems used in this process have greater demands placed on them. For instance, if the machine is running faster, pulp is required to be delivered to the core forming pocket quicker, necessitating a greater air flow to the pocket. To deliver more pulp to the pocket, more vacuum is required to retain the pulp within the pocket. One complication is in achieving optimum balance between air in to the pocket and vacuum applied to the back side of the pocket.

Imbalance between the amount of air supplying pulp to the core forming pocket and vacuum applied to the back of the pocket, holding the fluff in, causes puffs of fluff to escape forming chamber. Conventional core forming technology allows for limited adjustability to try and achieve the optimum balance between air in and vacuum. The largest air delivery is from fiberizing mill which supplies fluff and blows the fluff into the core forming chamber.

Another source of air into forming process is from the dust collection equipment, which returns collected fluff from the vacuum stream to the core forming drum. Beginning with fluff that passes through the core forming pocket, the vacuum stream leads the fluff within the vacuum stream to the dust collection unit. A filter within the dust collection unit captures this fluff, this fluff is removed from the filter, and recirculated into the core forming process. Typically, this vacuum stream is fed into a drum filter housing, such as described in U.S. Patent No. 5,679,136, commercial embodiments of which are available from the Osprey Corporation.

Pulpless cores are a recent development. In these embodiments, an absorbent core is formed without cellulosic fibers. It is known to use SAP with pockets created in a substrate (sometimes with vacuum), but formed pockets resist expansion with SAP expansion.

US 2003/120243 discloses an absorbent article comprising a liquid impermeable outer cover, a liquid permeable bodyside liner superposed on the outer cover and a continuous elastic absorbent core disposed between the outer cover and the bodyside liner.

US 2005/101929 discloses an absorbent core for a disposable absorbent article comprising a central fibrous layer and a three-dimensional sub-layer having a plurality of small pockets that contain superabsorbent polymer particles.

US 2006/167424 discloses a disposable absorbent article comprising a liquid permeable top-sheet, a liquid impermeable back-sheet and a pulp-less absorbent core interposed between the top-sheet and the back-sheet having a superabsorbent material.

US 2006/184149 discloses an absorbent article containing a web of hydrophilic fibres and a superabsorbent polymer embedded in the web.

### Summary of the Invention

The present invention provides a method of forming an absorbent core according to claim 1, a stretchable absorbent core according to claim 4 and a diaper product according to claim 7.

Also described is a method and apparatus for forming an absorbent core or cores. More particularly, the cores of the present invention, and methods for forming them, reduce or eliminate the absorbent fibrous material composed of cellulose wadding or cellulosic wood pulp material. The result is a pulpless core.

The method allows the ability to extend in a channel created along the machine direction if desired. Once the SAP expands, the corrugations (rugosity) can be elongated out, and wasted SAP is avoided if the SAP not exposed to liquid. Wasted SAP can be also avoided by a better control of SAP delivery, such as gradient deposition or pattern deposition. In the current art the acquisition/distribution layer (ADL) is responsible for all the liquid distribution, but in the present invention, the core itself can assist with even liquid retention, acquisition and distribution to the core.

A core is provided with flexibility for superabsorbent expansion by using elastic strands stretched and coupled to a preferably nonwoven layer, using elastic adhesive for example, and then the elastic/nonwoven combination is relaxed partially to still create receiving valleys (puckers) and these are filled with SAP and then a layer of nonwoven or acquisition film can be added on top of the elastic/nonwoven combination carrying SAP. The top nonwoven can be coupled to the elastic/nonwoven combination for instance by glue with core integrity adhesive.

In the present invention, the core preferably has some stretch in either the machine or cross-machine direction, which can assist with the fit of the product. Inextensible cores do not stretch, but the present invention provides for controlled amount and directional stretch as desired. The amount of stretch is fully adjustable by how much the elastic strands are initially stretched prior to coupling with a non-woven layer, and by how much the elastic/non-woven combination is relaxed. Additionally, directional stretch or patterned stretch can be provided by changing the elastic laydown pattern.

An absorbent core material is disclosed comprising a first material layer; a series of elongated elastic strand members running in a machine direction and spaced apart in a cross-machine direction, said elongated elastic members coupled to said first material layer at a first tension of said elongated elastic strand members; a superabsorbent polymer material provided in gaps between adjacent elongated elastic members at a second tension of said elongated elastic strand members, said first tension greater than said second tension; a second material layer coupled to said elongated elastic members; said first material layer, said elastic members, said suberabsorbent polymer and said second material layer creating a stretchable absorbent core web. The de-tensioning of the elastic creates valleys or depressions in the first material layer which can capture the superabsorbent material, and when the second material layer is provided and coupled to the first material layer, the stretchable absorbent core web is formed. A first zone comprising said first material layer, said elastic members, said suberabsorbent polymer and said second material layer is disclosed; and a second zone comprising said first material layer coupled directly to said second material layer, said second zone substantially free of suberabsorbent polymer is disclosed. After formation of the stretchable absorbent core web, the stretchable absorbent core web can be severed into discrete core portions, and placed onto a diaper chassis, or incorporated into a diaper product or a different type of absorbent disposable product as desired. The superabsorbent polymer material can be provided intermittently in said machine direction to create a machine direction superabsorbent polymer gap, and at this gap can be where said absorbent core material is severed into discrete core portions.

### Brief Description of the Drawings

Figs. 1a and 1b are in process top and side views respectively, of a core forming process of the present invention, with a first nonwoven web receiving a plurality of elastic strands under tension;
Figs. 2a and 2b are in process top and side views respectively, showing a first tension let off of the elastic strands, to create peaks and valleys in the nonwoven;
Figs. 3a and 3b are in process top and side views respectively, showing a SAP distribution system applying SAP laid into the created peaks and valleys in the nonwoven;
Figs. 4a and 4b are in process top and side views respectively, showing a second nonwoven layer applied over the elastic strands and SAP filled peaks and valleys in the first nonwoven layer;
Fig. 5 is a side view of the second nonwoven layer applied over the elastic strands and SAP filled peaks and valleys in the first nonwoven layer, showing a second tension let off of the elastic strands to create a continuous web of absorbent core material;
Fig. 6 is a first alternate embodiment of an elastic strand deposition pattern onto the first nonwoven layer;
Fig. 7 is a second alternate embodiment of an elastic strand deposition pattern onto the first nonwoven layer;
Fig. 8 is a cross sectional view of the second nonwoven layer applied over the elastic strands and SAP filled peaks and valleys in the first nonwoven layer, showing a second tension let off of the elastic strands, in an in use condition, for instance shaped as an absorbent core would be when a diaper is worn by a user;
Fig. 9 is a top view of the second nonwoven layer applied over the elastic strands and SAP filled peaks and valleys in the first nonwoven layer, showing a second tension let off of the elastic strands to create a continuous web of absorbent core material, the absorbent core rotated and placed on a receiver running web;
Fig. 10 is a top view of a portion of a system for producing an absorbent core;
Fig. 11 is a side view of the portion of the system shown in Fig. 10;
Fig. 12 is a side view of other portions of the system for producing an absorbent core shown in Fig. 10;
Fig. 13a is bottom view of a system for applying adhesive to a top nonwoven layer for use in creating an absorbent core;
Fig. 13b is a bottom view of an alternate system for applying adhesive to a top nonwoven layer for use in creating an absorbent core;
Fig. 13c is a top view of a SAP distribution system applying SAP in a preferred embodiment of SAP lanes to a base nonwoven layer coupled to elastic strands;
Fig. 13d is a top view of a SAP distribution system applying SAP in a second preferred embodiment of intermittent SAP lanes to a base nonwoven layer coupled to elastic strands;
Fig. 14 is a side view of a roller/nip combination coupling a top nonwoven layer to elastic strands carried by a base nonwoven layer, the base nonwoven layer carrying SAP material;
Fig. 15 is a cross sectional view of a top nonwoven layer coupled to elastic strands carried by a base nonwoven layer, the base nonwoven layer carrying SAP material to form an absorbent core laminate;
Fig. 16 is a side view of a system for winding a top nonwoven layer coupled to elastic strands carried by a base nonwoven layer, the base nonwoven layer carrying SAP material to form an absorbent core laminate;
Fig. 17 is a side view of a system for stacking a top nonwoven layer coupled to elastic strands carried by a base nonwoven layer, the base nonwoven layer carrying SAP material to form an absorbent core laminate;
Fig. 18 is a perspective view of an absorbent core laminate.

### Description of the Preferred Embodiment

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention.

In a preferred embodiment, creation of an absorbent core is accomplished by using two layers of nonwoven material 12 and 20, sandwiching SAP 16 and elastic strands 14 between the two layers of nonwoven material 12 and 20. However, in place of one or both of the nonwoven material layers 12 and 20, other materials can be used, such as woven materials, elastic materials, or any other useful material. The invention is not limited to use of nonwoven material layers in creating the laminate.

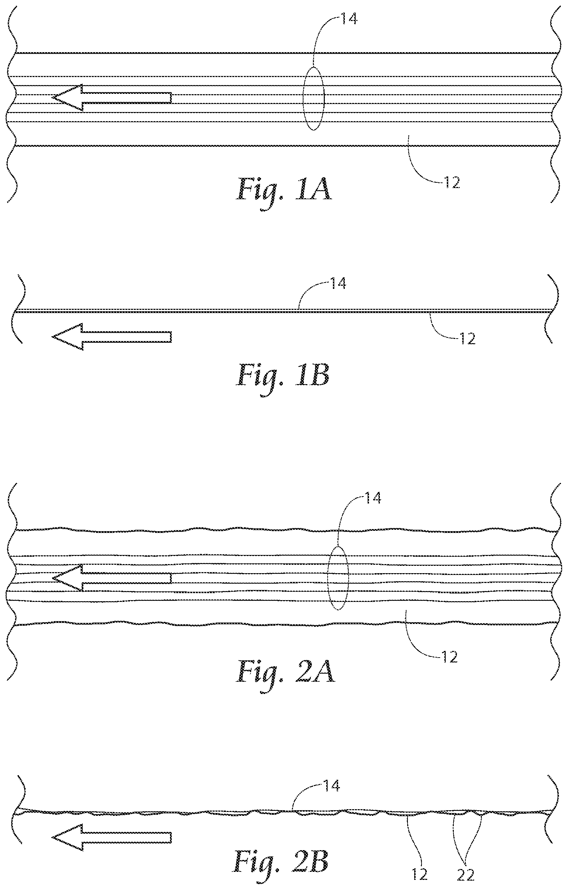

Referring now to Figs. 1a and 1b, in process top and side views respectively, of a core forming process are shown. A first nonwoven web 12 receives a plurality of elastic strands 14 under tension.

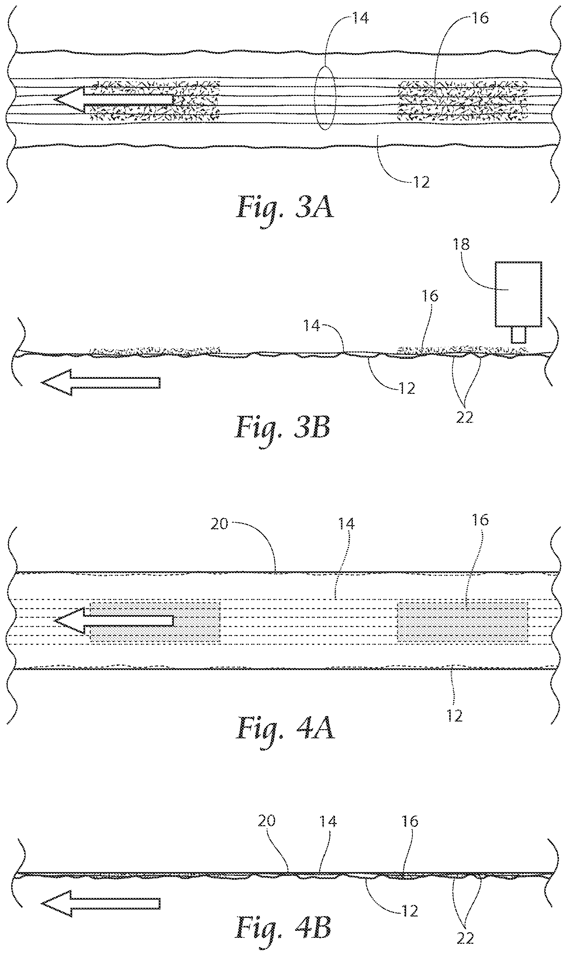

Referring now to Figs. 2a and 2b, a first tension let off of the elastic strands 14 is allowed, to create peaks and valleys (or puckers) 22 in the nonwoven 12. Referring now to Figs. 3a and 3b, a SAP distribution system 19 applies SAP laid 16 into the created peaks and valleys 22 in the nonwoven 12. The created peaks and valleys 22 act as pockets to carry the SAP 16. SAP 16 can be laid continuously (not shown) or discontinuously as shown. The SAP 16 delivery methods can be vibratory, by dimple drum, timed delivery, or any other methods known in the art. Additionally, the coordination between the SAP 16 deposition and the timing and orientation of joining nonwoven layer 20 can vary between in a vertical SAP 16 drop into a gap between vertically running web 20 and combined web 12/16 through nips, or the SAP 16 can be deposited onto horizontally machine direction running web 12/16 so that the pockets 22 can be collecting SAP 16 prior to horizontal machine direction running web 20.

The dimensions of the puckers 22 are adjustable with different spacing or number of strands of elastic 14. It may be desirable to vary the spacing and pucker size to allow higher or lower concentrations of SAP 16 in some sections of the diaper. The level of stretch in the strands 14 and the amount of relaxation before SAP 16 deposition can also change the pucker 22 dimensions.

In a preferred embodiment, SAP 16 can be deposited on nonwoven layer 12 while web 12 is moving horizontally in the machine direction, and when the second nonwoven layer 20 is applied and coupled, the combined pulpless core web can move downstream in any desired fashion.

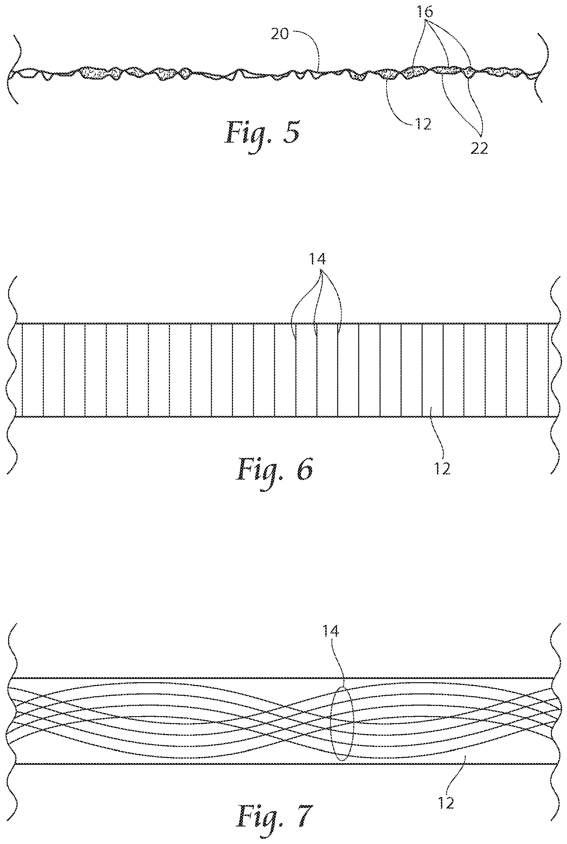

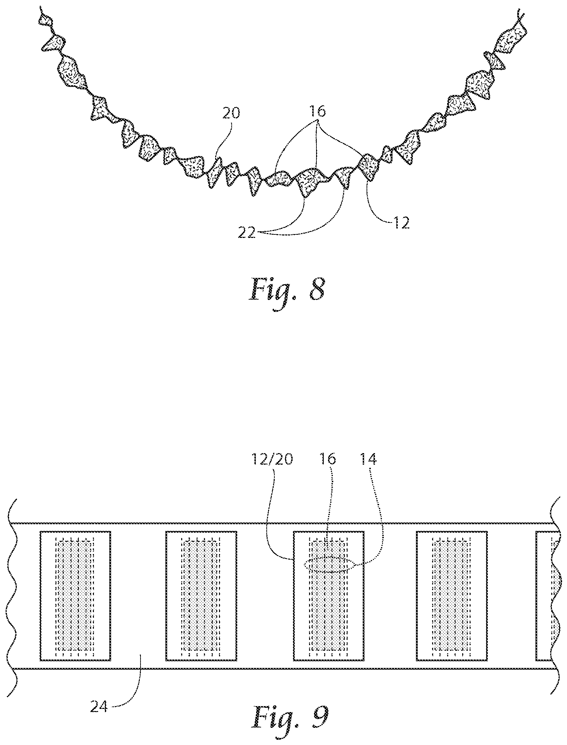

Referring to Figs. 4a and 4b, a second nonwoven 20 layer is applied over the elastic strands 14 and SAP 16 filled peaks and valleys 22 in the first nonwoven layer 12. As shown in Fig. 5, an alternate second tension let off of the elastic strands 14 create a continuous web of absorbent core material. The continuous web of absorbent core can then be processed as desired, for instance cut and placed onto a diaper chassis as individual cores, or rotated cut and placed as shown in Fig. 9 onto a running carrier web 24 which itself can be further processed as desired. The second nonwoven layer 20 could alternatively be a layer of acquisition/distribution film (an ADL layer). Describing an intended diaper from the outside, one preferred embodiment is a nonwoven layer, a poly layer, a nonwoven with lycra (core wrap) layer, SAP, an additional nonwoven (top core wrap), an acquisition/distribution layer, and a topsheet layer.

Referring still to Figs. 4a and 4b, SAP is shown deposited in areas where the elastic strands 14 reside, but outer channels of elastics 14 can be created that either do or do not contain SAP 16, as desired.

Figs. 6 and 7 show first and second alternate embodiments of elastic strand 14 deposition patterns onto the first nonwoven layer 12. In Fig. 6, the deposition is in the cross machine direction, and in Fig. 7, the deposition is in a curved fashion generally in the machine direction. The elastics 14 can be curved using curved or flared elastic deposition patterns as a method of tuning the absorption performance, and also to improve the fit and appearance of the final diaper product. The elastic deposition also provides control over the direction of elasticity of the core.

Referring to Fig. 8, a cross sectional view of the second nonwoven 20 layer applied over the elastic strands 14 and SAP 16 filled peaks and valleys 22 in the first nonwoven layer 12, shows a second tension let off of the elastic strands 14, in an in use condition, for instance shaped as an absorbent core would be when a diaper is worn by a user. It is evident that the rugosities desirably inhibit migration of SAP, for instance used beneficially in adult incontinent products. This feature could also increase the soft feel of the product, but in the current art, a thick ADL is needed to provide the soft feature of the product. The peaks and valleys or pockets 22 that are formed minimize the tendency for the SAP 16 to accumulate through gravity to crotch area, the lowest portion of the core as shown. The elastic strands and its lamination with nonwoven in the middle of the core can better immobilize the SAP on its wet stage.

The present invention may permit lower performance, less expensive, elastics to be used because the elastics and the cores formed thereby can hold a shape at body temperature. Less costly variations become possible e.g. lower decitex Spandex, plastomers or high copolymer olefins that are semi elastic. In place of or in addition to traditional elastics 14 as shown, ribbons, or films slit into ribbons or ribbonlike elements can be used in order to access these less costly technologies. The present invention can also use ultrasonic bonding to couple the layers (selecting Polypropylene as a blend component for example), or ultrasonic bonding can be used to trap elastic strands.

The entire crotch area can be provided with elasticity, and when combined with a front and back stretch panel, results in an overall diaper product which provides elastic from end to end of the diaper product if desired.

An additional embodiment (not shown), is that several layers of the absorbent core can be used. For instance, each layer might be designed for a different level of absorbency, or have different directional zones in it.

Additionally, a leg cuff could be built with into the core concept by stretching the outer strands of elastics 14 to a higher draw ratio than the central strands 14, and then when the product is allowed to spring back these strands of Lycra will raise up to make a bowl or boat shape without requiring a special apparatus and additional materials.

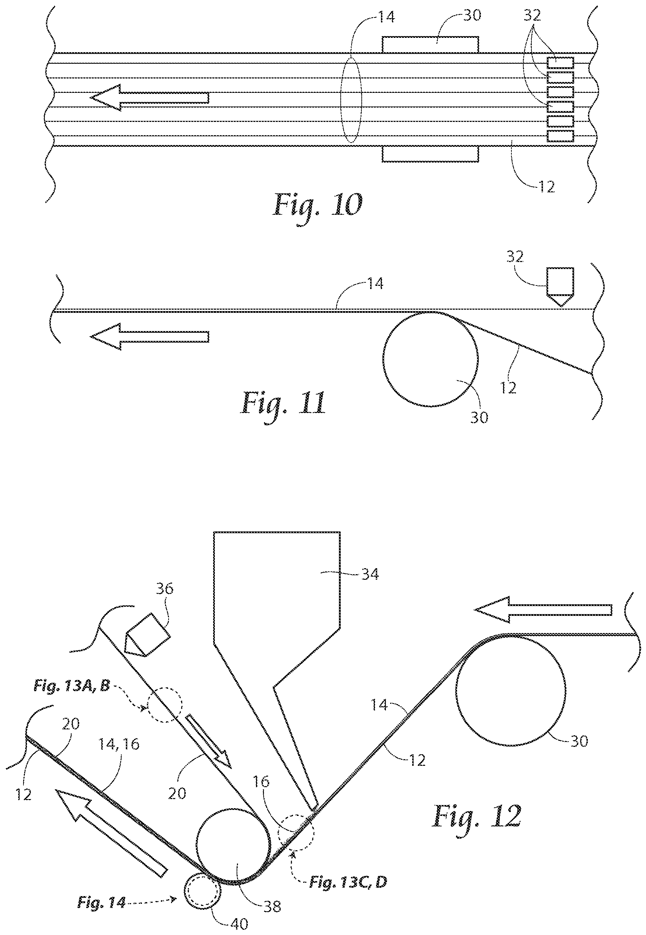

Referring now to Fig. 10, a top view (and in Fig. 11, a side view) of a portion of a system for producing an absorbent core laminate 12/14/16/20 is shown. Adhesive applicator or applicators 32 apply adhesive preferably to elastic strands 14 (or to nonwoven layer 12) and adhesive strands 14 are brought into contact with nonwoven layer 12 by passing the strands 14 and nonwoven layer 12 over a roller 30. Alternatively (not shown), a thermally compatible elastic 14 in strand or ribbon form can be used and ultrasonically bonded where desired.

Referring now to Fig. 12, after the elastic 14 is coupled to the base nonwoven 12, base nonwoven 12 is preferably slightly de-tensioned between roller 30 and roller 38/nip 40 combination to accommodate receipt of SAP 16 by the base nonwoven 12 by creating a slight furrow for SAP 16.

Draw ratios of elastic 14 can be varied. A larger draw ratio could reduce SAP 16 level per unit of nonwoven 12/elastic 14, but when relaxed, the SAP 16 can be contained in a relatively small area to achieve a high effective concentration of SAP. On the other hand, a smaller draw ratio will increase SAP level per unit of nonwoven 12/elastic 14. In this case, a higher SAP doping rate is used to achieve a high effective concentration of SAP. Higher draw ratio may desirably give a softer feel to the composite and when lower draw ratio can also reduce the cost of manufacturing. By changing the draw ratio, the performance and cost balance can be easily adjusted.

A SAP distribution system 34 applies SAP 16 to the base nonwoven 12. Incoming top nonwoven layer 20 receives adhesive from adhesive distribution system 36, and top nonwoven layer 20 is brought into contact with the base nonwoven 12 and elastic strand 14 combination at roller 38/nip 40 combination to create an absorbent core laminate 12/14/16/20 which is passed downstream for further processing as desired, for example cutting/placing, stacking, or rolling.

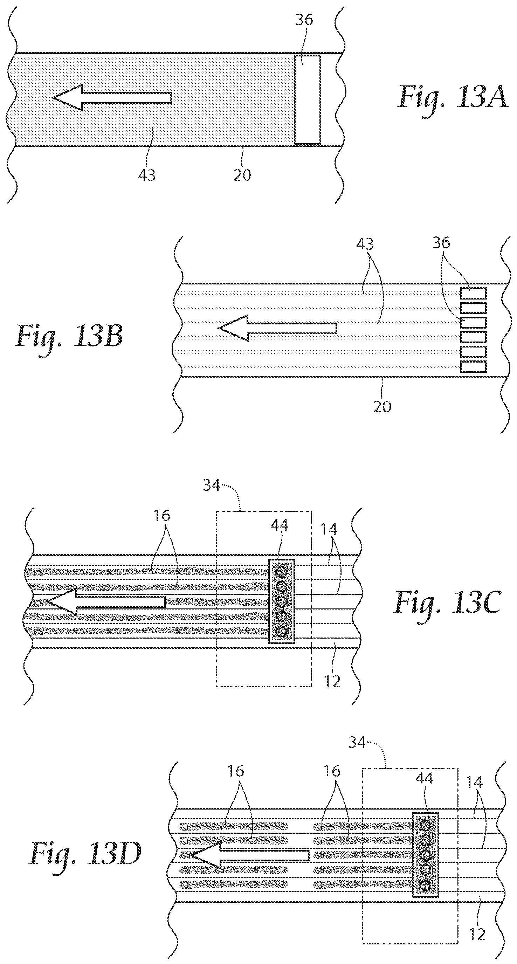

Referring now to Fig. 13a, a bottom view of adhesive applicator 36 is shown, applying adhesive 43 in a pattern to the bottom of top nonwoven layer 20. As shown in Fig. 13a, the pattern can be substantially continuous across desired areas of top nonwoven layer 20, or as shown in Fig. 13b, adhesive 43 can be applied in a striped pattern to top nonwoven 20. It may be desirable to apply adhesive 43 in a striped pattern to correlate to the spacing of elastic strands 14 carried by base nonwoven 12. As the spacing and orientation of elastic strands 14 carried by base nonwoven 12 can vary in spacing, orientation and laydown pattern, so too can the pattern of adhesive 43 applied to top nonwoven 20.

Referring now to Fig. 13c, a top view of a SAP distribution system 34 applying SAP 16 in a preferred arrangement of SAP 16 lanes to a base nonwoven layer 12 coupled to elastic strands 14 is shown. SAP 16 is received from a source such as a feed hopper as shown in Fig. 12, and passed to void spaces 44 in the SAP distribution system 34 to allow SAP 16 to be applied to the base nonwoven 12. A preferred SAP 16 laydown pattern is lanes or stripes as depicted in Fig. 13c, and as shown in Fig. 13d, these lanes can be intermittent SAP 16 lanes. Intermittent SAP 16 distribution is accomplished for example by interrupting the inbound flow of SAP 16, or by removing intermittently portions of the flow of SAP 16.

By forming puckers, channels or lanes in the core with SAP 16, retention, acquisition and distribution of liquid is assisted. In an alternative embodiment, alternating patterns or divergent patters of channels or lanes of SAP can be created by heavy and light SAP 16 distribution levels. This also enhances the distribution performance of the core and allows for the performance to be tailored according to the desired usage. In this manner, elastic strand 14 spacing can be alternated with heavy SAP 16 lanes adjacent to light SAP 16 lanes that can channel the liquid in a desired pattern.

The present invention allows for use of a thinner ADL layer because the contracted core is corrugated and can store liquid. The ADL layer can be allowed to contract with the absorbent core laminate 12/14/16/20 and the result is a fully elastic core in zones where elastification is desired.

For a higher dosage of SAP 16, larger (or longer) void spaces 44 and feed rate can be used. Likewise, for a lower dosage of SAP 16, smaller void spaces 44 and feed rates can be used.

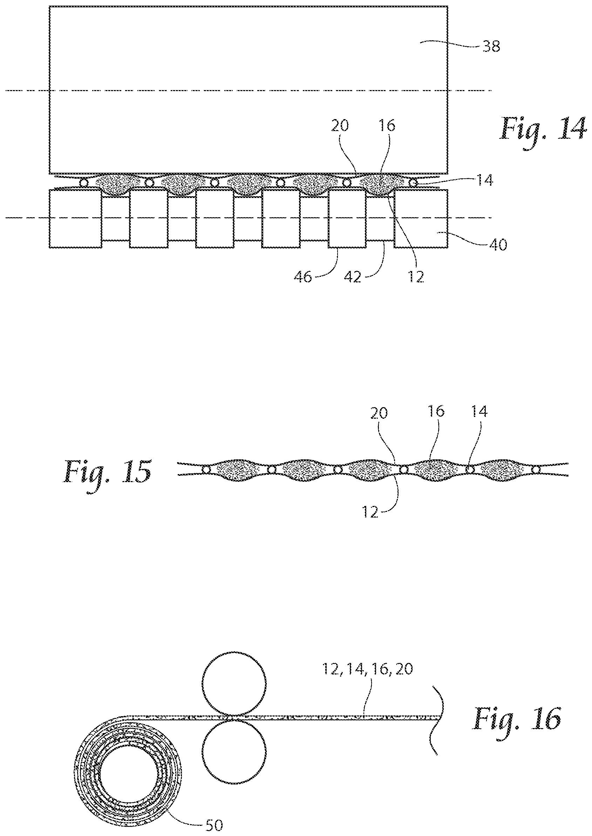

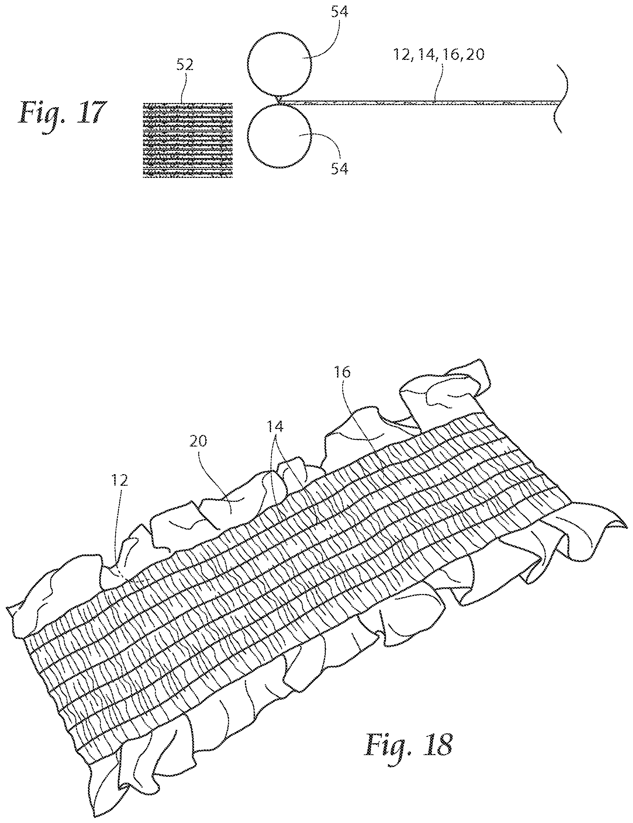

Referring now to Fig. 14, once SAP 16 has been applied to the base nonwoven layer 12, and adhesive 43 has been applied in a pattern to the bottom of top nonwoven layer 20, the materials of the absorbent core laminate 12/14/16/20 are brought together by nip wheel 38 urging the top nonwoven 20 carrying adhesive 43 against elastic strands 14. Base nonwoven 12 is carried by ridged nip wheel 40. In a preferred arrangement, peaks 46 of the ridged nip wheel 40 carry base nonwoven 12 and elastic strands 14, and the elastic strands 14 carried by base nonwoven 12 are urged into contact with top nonwoven 20. Valleys 42 of the ridged nip wheel 40 allow SAP 16 a position to migrate during compression of the materials of the absorbent core laminate 12/14/16/20 and coupling of the top nonwoven 20 to elastic strands 14. SAP 16 accumulates in the valleys 42. As shown in Fig. 15, an absorbent core laminate 12/14/16/20 is formed, with SAP 16 remaining captured in pockets, to create an absorbent core laminate 12/14/16/20 as shown in Fig. 18.

After creation of the absorbent core laminate 12/14/16/20, laminate is passed downstream for further processing as desired. This can include winding to create a roll 50 of absorbent core laminate 12/14/16/20 as shown in Fig. 16, cutting and stacking of absorbent core laminate 12/14/16/20 as shown in Fig. 17. Referring to Fig. 17 in particular, is a side view of a system for stacking a top nonwoven layer coupled to elastic strands carried by a base nonwoven layer, the base nonwoven layer carrying SAP material to form an absorbent core laminate is shown. A stack 52 of discrete absorbent core laminate 12/14/16/20 pieces can be assembled for packaging.

Preferably, absorbent core laminate 12/14/16/20 is cut and placed, and this cutting and placing can take any fashion such as traditional cores are variably placed in disposable product construction. For instance, the formed absorbent core laminate 12/14/16/20 can be situated adjacent to an acquisition/distribution (ADL) layer (not shown).

The foregoing is considered as illustrative only of the principles of the invention and it is not desired to limit the invention to the exact construction shown and described.

## Claims

1. A method of forming an absorbent core, the method comprising:
providing a first material layer (12);
coupling elastic strands (14) under tension to the first material layer (12) to form an elastic and material layer combination;
performing a first tension let-off to partially relax the elastic strands (14), wherein the partial relaxing of the elastic strands (14) deforms the first material layer (12) to form a plurality of valleys in the first material layer (12) for capturing a superabsorbent polymer material (16);
applying the superabsorbent polymer material (16) onto the first material layer (12); and
coupling a second material layer (20) to the elastic and first material layer combination.

2. The method of claim 1, wherein the superabsorbent polymer material (16) is applied when the first material layer (12) is moving horizontally in a machine direction.

3. The method of claim 1, wherein applying the superabsorbent polymer material (16) onto the first material layer (12) comprises applying the superabsorbent polymer material (16) in a plurality of lanes defined by adjacent pairs of elastic strands (14); and
wherein the plurality of lanes of the superabsorbent polymer material (16) are applied intermittently to form gaps in the superabsorbent polymer material (16) extending in the machine direction.

4. A stretchable absorbent core comprising:
a first material layer (12);
a series of elastic strand members coupled to the first material layer (12) at a partially tensioned state that deforms the first material layer (12) to form a plurality of valleys therein;
a second material layer (20) coupled to the elongated elastic strand members and over the first material layer (12); and
a superabsorbent polymer material (16) positioned in the plurality of valleys and between the first material layer (12) and the second material layer (20), so as to be captured in the plurality of valleys.

5. The stretchable absorbent core of claim 4, wherein the series of elastic strand members comprise linear elastic strands (14).

6. The stretchable absorbent core of claim 4, said absorbent core severed into discrete core portions.

7. A diaper product carrying a stretchable absorbent core portion of claim 6.

## Patentansprüche

1. Verfahren zum Herstellen eines absorbierenden Kerns, wobei das Verfahren Folgendes umfasst:
Bereitstellen einer ersten Materialschicht (12);
Koppeln von elastischen Strängen (14) unter Spannung an die erste Materialschicht (12), um eine Kombination aus elastischem Material und Materialschicht zu bilden;
Durchführen einer ersten Spannungsentlastung zum teilweisen Entspannen der elastischen Stränge (14), wobei das teilweise Entspannen der elastischen Stränge (14) die erste Materialschicht (12) verformt, um mehrere Vertiefungen in der ersten Materialschicht (12) zum Einfangen eines superabsorbierenden Polymermaterials (16) zu bilden;
Aufbringen des superabsorbierenden Polymermaterials (16) auf die erste Materialschicht (12); und
Koppeln einer zweiten Materialschicht (20) an der Kombination aus elastischem Material und erster Materialschicht.

2. Verfahren nach Anspruch 1, wobei das superabsorbierende Polymermaterial (16) aufgebracht wird, wenn sich die erste Materialschicht (12) horizontal in einer Maschinenrichtung bewegt.

3. Verfahren nach Anspruch 1, wobei das Aufbringen des superabsorbierenden Polymermaterials (16) auf die erste Materialschicht (12) das Aufbringen des superabsorbierenden Polymermaterials (16) in einer Mehrzahl von Bahnen, die durch benachbarte Paare elastischer Stränge (14) definiert sind, umfasst; und
wobei die mehreren Bahnen des superabsorbierenden Polymermaterials (16) intermittierend aufgebracht werden, um Lücken in dem superabsorbierenden Polymermaterial (16) zu bilden, die sich in Maschinenrichtung erstrecken.

4. Dehnbarer absorbierender Kern, umfassend:
eine erste Materialschicht (12);
eine Reihe von elastischen Strangelementen, die mit der ersten Materialschicht (12) in einem teilweise gespannten Zustand verbunden sind, welcher die erste Materialschicht (12) verformt, um mehrere Bahnen darin zu bilden;
eine zweite Materialschicht (20), die mit den länglichen elastischen Strangelementen verbunden und über der ersten Materialschicht (12) angeordnet ist; und
ein superabsorbierendes Polymermaterial (16), das in den mehreren Vertiefungen und zwischen der ersten Materialschicht (12) und der zweiten Materialschicht (20) angeordnet ist, um in den mehreren Vertiefungen eingefangen zu werden.

5. Dehnbarer absorbierender Kern nach Anspruch 4, wobei die Reihe von elastischen Strangelementen lineare elastische Stränge (14) umfasst.

6. Dehnbarer absorbierender Kern nach Anspruch 4, wobei der absorbierende Kern in separate Kernabschnitte getrennt ist.

7. Windelprodukt, das einen dehnbaren absorbierenden Kernteil nach Anspruch 6 aufweist.

## Revendications

1. Procédé pour former un noyau absorbant, le procédé consistant à :
fournir une première couche matérielle (12) ;
coupler des fils élastiques (14) sous-tension à la première couche matérielle (12) pour former une combinaison de couches élastiques et matérielles ;
effectuer un premier relâchement de tension pour relâcher partiellement les fils élastiques (14), le relâchement partiel des fils élastiques (14) déformant la première couche matérielle (12) pour former une pluralité de vallées dans la première couche matérielle (12) pour capturer un matériau polymère superabsorbant (16) ;
appliquer le matériau polymère superabsorbant (16) sur la première couche matérielle (12) ; et à
accoupler une seconde couche matérielle (20) à la combinaison de premières couches élastiques et matérielles.

2. Procédé selon la revendication 1, dans lequel le matériau polymère superabsorbant (16) est appliqué quand la première couche matérielle (12) se déplace horizontalement dans la direction de la machine.

3. Procédé selon la revendication 1, dans lequel appliquer le matériau polymère superabsorbant (16) sur la première couche matérielle (12) consiste à appliquer le matériau polymère superabsorbant (16) dans une pluralité de chemins définis par des paires adjacentes de fils élastiques (14) ; et
dans lequel la pluralité de chemins du matériau polymère superabsorbant (16) sont appliqués par intermittence pour former des creux dans le matériau polymère superabsorbant (16) s'étendant dans la direction de la machine.

4. Noyau absorbant étirable comprenant :
une première couche matérielle (12) ;
une série d'éléments fils élastiques couplés à la première couche matérielle (12) à un état partiellement tendu qui déforme la première couche matérielle (12) pour y former une pluralité de vallées ;
une seconde couche matérielle (20) couplée aux éléments fils élastiques allongés et sur la première couche matérielle (12) ; et
un matériau polymère superabsorbant (16) positionné dans la pluralité de vallées et entre la première couche matérielle (12) et la seconde couche matérielle (20) de façon à être capturé dans la pluralité de vallées.

5. Noyau absorbant étirable selon la revendication 4, dans lequel la série d'éléments fils élastiques comprend des fils élastiques linéaires (14).

6. Noyau absorbant étirable selon la revendication 4, ledit noyau absorbant étant coupé en portions de noyau discrètes.

7. Couche portant une portion de noyau absorbant étirable selon la revendication 6.
